(19) Europäisches Patentamt
European Patent Office
Office européen des brevets

(11) **EP 2 928 577 B1**

(12) **EUROPEAN PATENT SPECIFICATION**

(45) Date of publication and mention
of the grant of the patent:
**05.07.2023 Bulletin 2023/27**

(21) Application number: **13863417.5**

(22) Date of filing: **10.12.2013**

(51) International Patent Classification (IPC):
**B01D 61/14** (2006.01)   **B01D 39/16** (2006.01)
**B01D 67/00** (2006.01)   **B01D 71/56** (2006.01)
**B01D 69/06** (2006.01)   **A61L 2/02** (2006.01)

(52) Cooperative Patent Classification (CPC):
**B01D 71/56; A61L 2/022; B01D 39/1623;**
**B01D 61/145; B01D 67/0004; B01D 69/06;**
B01D 2239/025; B01D 2239/0631;
B01D 2239/0654; B01D 2239/1233; B01D 2323/39

(86) International application number:
**PCT/US2013/074132**

(87) International publication number:
**WO 2014/093345 (19.06.2014 Gazette 2014/25)**

(54) **ULTRAPOROUS NANOFIBER MATS AND USES THEREOF**

ULTRAPORÖSE NANOFASERMATTEN UND VERWENDUNGEN DAVON

MATS DE NANOFIBRES ULTRAPOREUX ET LEURS UTILISATIONS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.12.2012 US 201261735275 P**

(43) Date of publication of application:
**14.10.2015 Bulletin 2015/42**

(73) Proprietor: **EMD Millipore Corporation
Burlington, MA 01803 (US)**

(72) Inventors:
• **KOZLOV, Mikhail
Billerica, MA 01821 (US)**
• **TKACIK, Gabriel
Billerica, MA 01821 (US)**
• **KAS, Onur, Y.
Billerica, MA 01821 (US)**
• **POTTY, Ajish
Billerica, MA 01821 (US)**

• **PATEL, Jigneshkumar
Billerica, MA 01821 (US)**

(74) Representative: **Graham Watt & Co LLP
St. Botolph's House
7-9 St. Botolph's Road
Sevenoaks TN13 3AJ (GB)**

(56) References cited:
**US-A1- 2006 016 748      US-A1- 2008 164 214
US-A1- 2008 264 259      US-A1- 2011 198 282
US-A1- 2011 198 282      US-A1- 2012 061 332
US-A1- 2012 061 332      US-A1- 2012 091 072
US-A1- 2012 125 847**

• **Hongyang Ma ET AL: "Ultra-fine cellulose
nanofibers: new nano-scale materials for water
purification", Journal of Materials Chemistry, vol.
21, no. 21, 1 January 2011 (2011-01-01), page
7507, XP055677844, GB ISSN: 0959-9428, DOI:
10.1039/c0jm04308g**

**Description**

Field of the Invention

The present invention relates generally to liquid filtration media. The invention provides methods of preparing nanofiber polymeric materials having extremely small fiber diameters, assembling these fibers into highly consistent mats, and using such mats for removal of viruses from fluid streams.

Background of the Invention

Regulatory agencies around the world place stringent requirements on commercial manufacturers of biopharmaceutical compounds to provide biosafety assurance of their drugs. The manufacturers have to build in and validate at least two orthogonal (operating by two distinct mechanisms) steps of virus removal into their processes, one of which is usually size-based filtration. The expected LRV (log reduction value) of the filtration is at least 4.

Current strategies in viral filtration are provided in Meltzer, T., and Jornitz, M., eds., "Filtration and Purification in the Biopharmaceutical Industry", 2nd ed., Informa Healthcare. 2008, Chapter 20, "Ensuring Safety of Biopharmaceuticals: Virus and Prion Safety Considerations", H. Aranha.

Parvoviruses, non-enveloped icosahedral particles of 18 to 26 nm in size, are some of the smallest known viruses (Leppard, Keith; Nigel Dimmock: Easton, Andrew (2007). Introduction to Modem Virology. Blackwell Publishing Limited, p. 450). Manufacturers of virus-retentive membrane routinely rely on measurements of parvovirus retention for validation of virus removal assurance of their membranes.

There are a number of commercially available membranes validated for parvovirus removal. An exemplary parvovirus removal membrane, Viresolve® Pro, available from EMD Millipore Corporation, Billerica, MA USA, has an asymmetrical pore structure, with a tight virus removal side and microporous "support" side. It is manufactured by a phase inversion process used to make a wide range of ultra- and microfiltration membranes. An inherent limitation with the phase inversion manufacturing process is that membrane porosity decreases significantly with pore size.

For example, a microporous membrane with an average pore size of 0.5 micron may have porosity of about 75-80%, while an ultrafiltration or virus removal membrane with an average pore size of 0.01 micron to 0.02 micron will only be less than 5% porous in its region of narrowest pore size. Thus, the parvovirus removal membranes have traditionally low porosity and thus lower water flux.

As biopharmaceutical manufacturing becomes more mature, the industry is constantly looking for ways to streamline the operations, combine and eliminate steps, and reduce the time it takes to process each batch of the drug. At the same time, there are market and regulatory pressures requiring manufacturers to reduce their costs. Since virus filtration accounts for a significant percentage of the total cost of drug purification, any approach to increase membrane throughput and reduce drug processing time would be valuable. With the introduction of new pre-filtration media and corresponding increase in throughput of virus filters, filtration of more and more feed streams is becoming flux-limited. Thus, improvements in the permeability of virus filters, while maintaining the virus filters' virus retention properties will have a direct effect on the cost of virus filtration step.

Electrospun nanofiber mats are highly porous polymeric materials, wherein the "pore" size of the mat is linearly proportional to the fiber diameter of the electrospun nanofiber, while the porosity of the mat is relatively independent of the fiber diameter and usually falls in the narrow range of 85-90%. Such high porosity is responsible for the substantial improvement of permeability provided in electrospun nanofiber mats when compared to the porosity of immersion cast membranes of similar thickness and pore size rating. Moreover, this advantage becomes amplified in the smaller pore size range, such as those required for virus filtration, because of the reduced porosity of ultrafiltration membranes discussed *supra*.

The random nature of electrospun mat formation has led to the general assumption that such mats are unsuitable for any critical filtration of liquid streams. Applications of electrospun materials for the reliable removal of relatively large particles (such as bacteria) from solutions have recently begun to appear in the literature (See, for example, International Publication No. WO2010/107503 A1, to EMD Millipore Corporation, titled "Removal of Microorganisms from Fluid Samples Using Nanofiber Filtration Media", and Wang et al., "Electrospun nanofibrous membranes for high flux microfiltration", Journal of Membrane Science, 392-393 (2012) 167-174). At the same time, no reports have been published on using electrospun nanofibers for size-based filtration of extremely small particles, such as parvoviruses.

US2012/0125847A1 discloses a method for removing pathogens from a transfusion grade platelet composition. US2006/0016748A1 discloses a hydrophilic microporous membrane comprising a thermoplastic resin. US2012/0061332A1 discloses a method for removing retroviruses from liquid samples and a nanofiber containing liquid filtration medium. Ma et al. J.Mater. Chem., 2011, 21, 7507-7510 discloses Ultra-fine cellulose nanofibers for water purification.

Three categories of prior art pertaining to virus removal and electrospun nanofibers can be generally are described as follows:

Category 1. Virus removal using electrospun materials by adsorption or inactivation

US Published Patent Application No. US2008/0J64214 A1 to Advanced Powder Technologies, teaches a liquid purification and disinfection nonwoven filter material characterized by the electrostatic sorption of contaminants, including electronegative particles, e.g. bacteria, viruses, colloidal particles, etc.

US Patent No. 6,770,204 to Koslow Technologies Corporation, teaches a composite filter medium having a pH altering material that can raise the pH of an influent such that microbiological contaminants in the influent remain substantially negatively charged, such that a positively charged medium within the composite filter medium can more effectively capture the microbiological contaminants.

US Patent No. 7.927.885 to Fujifilm Corp., provides electrospun support material carrying antibodies for virus removal.

US Published Patent Application No. US2008/0264259, assigned to The Hong Kong Polytechnic University, and titled "Nanofiber Filter Facemasks And Cabin filters", teaches a filtration medium including a fine filter layer having a plurality of nanofibers and a coarse filter layer having a plurality of microfibers attached to the fine filter layer, wherein the nanofibers comprise an electrical charge or anti-microbial agent.

International Publication No. WO2008/073507 to Argonide Corp., teaches a fibrous structure for fluid streams comprising a mixture of nanoalumina fibers and additional fibers made from microglass, cellulose, fibrillated cellulose, and lyocell, and arranged in a matrix to create asymmetrical pores and to which fine. ultrafine, or nanosize particles such as powdered activated carbon are attached without the use of binders. The fibrous structure containing powdered activated carbon intercepts contaminants (such as viruses) from fluid streams.

The filter materials within Category 1 appear to take advantage of certain surface effects of the electrospun media, either by adsorbing or inactivating viruses,

Category 2. Microorganism removal by electrospun materials using sieving mechanisms.

International Publication No. WO2010/107503 to EMD Millipore Corporation teaches a method for highly efficient size-based removal of bacteria and mycoplasma from liquid samples using electrospun nanofibers.

International Publication No. WO2012/021308 to EMD Millipore Corporation teaches size-based removal of retroviruses (having 80-130 nm) with LRV >6 using electrospun nanofiber mats.

US Published Patent Application No. IJS2011/0198282 to State University of New York Research Foundation, titled "High Flux High Efficiency Nanofiber Membranes and Methods of Production Thereof, teaches composite nanofiber membranes comprising an electrospun substrate coated with cellulose nanofibers, produced from oxidized cellulose microfibers layer, is applied thereto.

US Published Patent Application No. US2008/0264258 to Elmarco SRO teaches a filter for removing physical and/or biological impurities comprising an "active" nanofiber filter that purportedly kills/weakens impurities, while a nanofiber filtration layer captures the impurities.

A series of US Published Patent Applications Nos. US2004/0038014. US2005/0163955, and US2004/0038013, each assigned to Donaldson, Inc.. teach fiber-containing media, and fiber mats having 30 nm fibers treated with temperature and pressure.

A conference report from Nanocon 2010, by Lev et al., teaches using commercial nanofiber fabrics with fiber diameters between ~100 nm to 155 nm to retain E. Coli bacteria (1.1 - 1.5 x 2 microns to 6 microns) with efficiency 72.25% to 99.83% (0.6 to 2.8 LRV).

International Publication No. WO2009/071909. assigned to Munro Technology Ltd., teaches a spatially-ordered matrix array of nanometre fibres with nanometre-size voids, suitable for filtration of particles, in particular particles in the nanometer-size range, such as viruses. However, there are no examples provided showing successful filtration.

None of the filter materials in Category 2 appear to enable size-based removal of viruses or particles under 30 nm in size.

Category 3. Attempts to reduce fiber diameter below 20 mn.

Huang et al.. Nanotechnology 17 (2006) 1558-1563, teaches electrospun polymer nanofibres having a small diameter, and providing therein microscopic observations of individual fibers as small as 2 nm, produced using 2% Nylon 4,6 with added pyridine,

US Patent No. 7,790,135 assigned to Physical Sciences, Inc., teaches a method of electrospinning polyacrylonitrile fibers as small as 15 nm and subsequent pyrolysis to produce a carbon nanotube mat out of therefrom.

Tan et al., Polymer 46, (2005) 6128-6134, offers a systematic parameter study for ultra-fine fiber fabrication via electrospinning process, including the fabrication of a mat having average fiber diameters of $19\pm6$ nm.

Hou et al., Macromolecules 2002, 35, 2429-2431, teach poly(p-xylylene) nanotubes by coating and removal of ultrathin polymer template. Individual fibers observed with diameters 5-7 nm.

Duan et al., 2008 2nd IEEE International Nanoelectronics Conference (INEC 2008), 33-38, teach preparing graphitic nanoribbons from ultrathin electrospun PMMA (polymethyl methacrylate) nanofibers by electron beam irradiation, wherein individual PMMA fibers were to have observed average diameters around 10 nm.

Each of the electrospun nanofiber teachings in Category 3 attempt to reduce the fiber diameter of electrospun materials. While certain electrospun nanofiber teachings in Category 3 allege individual fibers as small as 10 nm in diameter or less, at best these teachings provide microscopic images of a single fiber of unknown length, and fail to provide any data on obtaining or systematically attempting consistent fiber mats with the average size of all fibers in the mats in the 6 nm to 13 nm range. In particular, no such mats have been reported indicating any capability of currently known virus-retentive membranes.

The present invention provides an electrospinning-based method to manufacture very fine nanofiber mats with exceptionally high uniformity, and for use in reliably and efficiently removing viral particles from fluid streams. As provided herein high retention of model parvovirus (> 3 LRV) is accomplished with a polymeric nanofiber electrospun porous liquid filtration mat. These electrospun nanofiber mats can be used for virus removal from aqueous solutions in hiopharmaceutical manufacturing, where these electrospun nanofiber mats have an advantage of high permeability and high capacity compared to the state of the art current viral removal filtration products.

Summary of the Invention

The present invention teaches highly porous electrospun filtration membranes having very small pore sizes that can be used for the retention of parvoviruses and other particles in the 18 nm to 30 nm size range. The high porosity electrospun filtration membranes provided herein enable higher water fluxes. thus substantially reducing the amount of time required for virus filtration steps. With this increased speed of virus filtration, or, alternatively, lower pressures as required for the same operation, electrospun parvovirus filters such as taught herein enable applications of virus filtration not previously believed known.

For example, a lower pressure requirement can enable using virus-removal filters with simpler, less costly and complicated equipment, such as gravity flow holders, and vacuum and peristaltic pumps. Also, higher permeability allows for the economical filtration of large fluid volumes during protein purification processes, such as the entire volume of a bioreactor and process buffer solution, thereby creating a virus "barrier" around the entire protein purification process.

The present invention is based, at least in part, on the surprising discovery that a previously unknown combination of spinning solution parameters and environmental conditions results in the manufacture of highly consistent electrospun mats having extremely small effective pore sizes. The term "effective pore size", as used herein, describes a *structural* property of a porous material assessed with a *functional,* rather than visual, method. For the purposes of comparing porous materials with dramatically different structures, such as solution-cast membranes and nanofiber mats, visual methods like microscopy are inadequate in predicting whether these materials would be perform similarly in the same application. In contrast, functional methods, such as bubble point measurements, liquid-liquid porometry. intrusion porosimetry, sieving of macromolecules and/or particles of given sizes, allow to compare the properties of different materials. Titus, comparisons are possible between different materials, which can be described having "smaller", "larger", or "similar" effective pore sizes depending on how they perform in a functional test.

In some embodiments of the present invention, electrospun nanofibers are produced having an average diameter between 6 nm and 13 nm.

In other embodiments, the nanofibers are assembled into consistent mats having a mean flow bubble point measured with perfluorohexane fluid above 100 psi, or above 120 psi. or above 130 psi (1 psi = 6.89 kPa).

In some embodiments, the nanofiber mats can be assembled in single or multilayered devices for liquid filtration.

In some embodiments, according to the various aspects of the present invention, an aqueous feed solution containing a model or actual parvovirus can be filtered with a device containing electrospun nanofiber mats as taught herein, so that in one embodiment the filtrate will contain less than 0.1% of viruses present in the feed solution; in other embodiments the filtrate will contain less than 0.01% of viruses present in the feed solution; or in other embodiments less than 0.001% of viruses present in the feed solution; or in other embodiments the filtrate will contain less than 0.0001% of viruses present in the feed solution.

In some embodiments, solutions intended for virus filtration contain a biopharmaceutical product of interest, such as therapeutic protein, antibody, hormone, or the like.

In other embodiments, solutions intended for virus filtration are aqueous buffer solutions.

In yet other embodiments, solutions intended for virus filtration are liquid media for cell culture bioreactor.

In some embodiments according to the various aspects of the present invention, the methods and/or compositions of the present invention may be used in combination with one or more other filtration, clarification, and pre-filtration steps.

Additional features and advantages of the invention will be set forth in the detailed description and claims, which follows. Many modifications and variations of this invention can be made without departing from its scope, as will be apparent to those skilled in the art. It is to be understood that the foregoing general description and the following detailed description, the claims, as well as the appended drawings are exemplary and explanatory only, and are intended to provide an explanation of various embodiments of the present teachings. The specific embodiments described herein are offered by way of example only and are not meant to be limiting in any way.

BRIEF DESCRIPTION OF THE DRAWINGS

The accompanying drawings, which are incorporated in and constitute a part of this specification, illustrate the presently contemplated embodiments of the invention and, together with the description, serve to explain the principles of the invention.

Figure I depicts a SEM micrograph of an electrospun mat obtained with 8% nylon 6. 40% 2,2.2-trifluoroethanol (TFE) and 0.7% ammonium formate in the mix in accordance with an embodiment of this invention.

Figures 2A and 2B each depict SEM micrographs of electrospun mats produced with the same solution at 4° C dew point (left, fiber diameter 23 nm) and 17° C dew point (right, fiber diameter 9 nm) in accordance with other embodiments of this invention.

Figures 3A and 3B each depict SEM micrographs of improvements to nylon electrospun mats quality when 2.2,2-trifiuroethanol (TFE) is added to the spinning mixture (right), according to Examples 2A and 2B, in accordance with other embodiments of this invention.

Figures 4A, 4B, 4C, and 4D each depict SEM micrographs of electrospun mats wherein the nylon 6 concentration in the spinning solution according to Example 3 is reduced from 14%wt. to 8%wt., resulting in the formation of highly irregular mats.

Figure 5 depicts SEM micrographs of an electrospun mats obtained with standard 8% nylon 6 mix and with 7% nylon 6 mix.

Figure 6 depicts a graphical representation of PhiX-174 LRV vs. throughput for three (3) layer devices manufactured according to Example 3, average of two (2) devices. The assay limit is about 6.3 LR V .

Figure 7 depicts a graphical representation of dextran retention curves for Viresolve™ Pro membrane, and an electrospun mat in accordance with other embodiments of this invention.

Figure 8 depicts PhiX-174 retention (open symbols and dotted lines) and flux decay (closed symbols and solid lines) for one (1) layer of Viresolve® Pro membrane (triangles) and three (3) layers of electrospun composites (squares) in accordance with other embodiments of this invention, at 30 psi pressure.

DETAILED DESCRIPTION OF EMBODIMENTS OF THE INVENTION

Before describing the present invention in further detail, a number of terms will be defined. Use of these terms does not limit the scope of the invention but only serve to facilitate the description of the invention. Additional definitions are set forth throughout the detailed description.

I. DEFINITIONS

As used herein, the singular forms "a," "an," and "the" include plural referents unless the context clearly dictates otherwise.

As used herein, the term "nanofibers" refers to fibers having diameters varying from a few nanometers up to several hundred nanometers.

As used herein, the terms "filter medium" or "filter media" refer to a material, or collection of material, through which a fluid carrying a microorganism contaminant passes, wherein the microorganism is deposited in or on the material or collection of material.

As used herein, the terms "permeability" refers to the rate at which a volume of fluid passes through a filtration medium of a given area at a given pressure drop across the membrane. Common units of permeability are Liters per square meter per hour for each psi of pressure drop, abbreviated as LMH/psi where 1 psi=6.89 kPa.

The term "electrospinning", as used herein, refers to an electrostatic spinning process of producing nanofibers from a polymer solution or melt by applying an electric potential to such solution. The electrostatic spinning process for making an electrospun nanofiber mat for a filtration medium, including a suitable apparatus for performing the electrostatic spinning process is disclosed in International Publication Nos. WO 2005/024101, WO 2006/131081, and WO 2008/106903, each assigned to Elmarco S.R.O,. of Liberec Czech Republic.

The term "nanofiber mat" as used herein, refers to an assembly of multiple nanofibers, such that the thickness of said mat is at least about 10 times greater than the diameter of a single fiber in the mat. The nanofiber can be arranged randomly in the said mat, or be aligned along one or multiple axes.

The term "virus" as used herein, refers to a small infectious agent that can replicate only inside the living cells of an organism. Viruses can infect both eukaryotic and bacterial cells. While the former are relevant for ensuring the safety of therapeutic formulations, the latter are a common surrogate used to assess retention properties of virus removal filters.

As used herein, the term "parvovirus", refer to a class of some of the smallest, non-enveloped icosahedral particles of 18 nm to 26 nm in size (Leppard, Keith; Nigel Dimmock; Easton, Andrew (2007). Introduction to Modern Virology. Blackwell Publishing Limited p. 450).

The term "LRV", or "Logarithmic Reduction Value", as used herein, refers to a common logarithm (base 10) of the ratio of particle concentration in the feed to that in filtrate, measured under standardized conditions.

The term "immunoglobulin." "Ig" or "antibody" (used interchangeably herein) as used herein refers to a protein having a basic four-polypeptide chain structure consisting of two heavy and two light chains, said chains being stabilized, for example, by interchain disulfide bonds, which has the ability to specifically bind antigen.

As used herein, immunoglobulins or antibodies may be monoclonal or polyclonal and may exist in monomeric or polymeric form, for example. IgM antibodies which exist in pentameric form and/or IgA antibodies which exist in monomeric, dimeric or multimeric form. The term "fragment" refers to a part or portion of an antibody or antibody chain comprising fewer amino acid residues than an intact or complete antibody or antibody chain. Fragments can be obtained via chemical or enzymatic treatment of an intact or complete antibody or antibody chain. Fragments can also be obtained by recombinant means. Exemplary fragments include Fab, Fab', F(ab')2, Fc and/or Fv fragments.

The term "biopharmaceutical preparation." as used herein, refers to any composition containing a product of interest (e.g.. a therapeutic protein or an antibody, which is usually a monomer) and unwanted components, such as protein aggregates (e.g.. high molecular weight aggregates of the product of interest).

## II. EXEMPLARY FILTRATION MEDIUM

An embodiment of the present invention includes a porous electrospun nanofiber liquid filtration mat.

An additional embodiment of the invention includes a liquid filtration medium having nanofibers with an average fiber diameter of 6 nm to 13 nm, wherein the filtration medium has a mean pore size ranging from 0.01 $\mu$m to 0.03 $\mu$m, a porosity ranging from 80% to 95%, a thickness ranging from 1 $\mu$m to 100 $\mu$m, or a thickness from about 2 $\mu$m and about 30 $\mu$m, and a liquid permeability greater than 1.45 LMH/kPa (10 LMH/psi).

## III. EXEMPLARY NANOFIBER POLYMERIC MATERIALS

Polymers suitable for use in nanofibers include thermoplastic and thermoset polymers. Examples of suitable polymers not falling under the scope of the claims include polyimide, aliphatic polyamide, aromatic polyamide, polysulfone. cellulose, cellulose acetate, polyether sulfone, polyurethane, poly(urea urethane), polybenzimidazole, polyetherimide, polyacrylonitrile, poly(ethylene terephthalate), polypropylene. polyaniline, poly(ethylene oxide), poly(ethylene naphthalate). poly(butylene terephthalate), styrene butadiene rubber, polystyrene, poly(vinyl chloride), poly(vinyl alcohol), poly(vinyl acetate), poly(vinylidene fluoride), poly(vinyl butylene), copolymers, derivative compounds, blends and combinations thereof. Suitable polyamide condensation polymers, falling under the scope of the claims, include nylon-6; nylon-6,6;nylon 6,6-6,10; copolymers of the same.

The term "nylon" as used herein includes nylon-6, nylon-6,6, nylon 6.6-6.10. and copolymers, blends and combinations thereof.

## IV. EXEMPLARY METHODS OF FORMING A FIBROUS MAT

In one embodiment of the invention, a fibrous mat is made by depositing nanofiber(s.) from a nylon solution. The nanofiber mat has a basis weight of between about 0.1 g/m$^2$ and about 10 g/m$^2$, as measured on a dry basis, (i.e.. after the residual solvent has evaporated or otherwise been removed).

In another embodiment of the invention, nylon is dissolved in a mixture of solvents including, but not limited to. formic acid, sulfuric acid, acetic acid. 2,2,2-trifluoroethanol,2,2,2,3,3,3-hexafluoropropanol, and water.

In another embodiment of the invention, the nylon solution is prepared by dissolving dry nylon polymer in one group of solvents, i.e. first preparing a stock solution, and then adding other solvents to make the solution ready for electrospinning.

In another embodiment of the invention, the nylon polymer (i.e., starting) is partially hydrolyzed over the course of solution preparation, such that the average molecular weight of the partially hydrolyzed nylon polymer (i.e., ending) is less than the average molecular weight of the starting nylon polymer.

In an additional embodiment of the invention, conductivity of the nylon solution is adjusted with a suitable ionizable compound in a given solvent. Examples of such suitable ionizable compounds include, but are not limited to, organic and inorganic salts, acids and bases. An example of a preferred compound used to adjust the conductivity of a nylon solution is ammonium formate.

In another embodiment of the invention, the environment inside the electrospinning chamber is controlled to ensure that ambient humidity is kept at dew point above approximately 12° C.

In one embodiment of the invention, a variety of porous single or multilayered substrates or supports are arranged on a moving or stationary collection belt to collect and combine with the electrospun nanofiber mat medium, forming a composite filtration device.

## V. EXEMPLARY SUBSTRATES FOR COLLECTING THE NANOFIBERS

Examples of single or multilayered porous substrates or supports include, but are not limited to, spunbonded nonwovens, meltblown nonwovens, needle punched nonwovens, spunlaced nonwovens, wet laid nonwovens, resin-bonded nonwovens, woven fabrics, knit fabrics, paper, and combinations thereof, as well as other electrospun mats with fibers of similar or greater diameter to the electrospun nanofibers collected thereon.

In one embodiment of the invention, when the substrate layer is a nanofiber mat, the average fiber diameter of the substrate can range from 10 nm to 500 nm.

In another embodiment, average fiber diameter of the substrate can range from 50 nm to 200 nm.

In another embodiment, average fiber diameter of the substrate can range from 10 nm to 50 nm.

An important consideration for choosing a substrate layer is the smoothness of the surface, in other words, the diameter of the substrate fibers relative to the diameter of the fibers making up the active nanofiber layer. As described in WO 20 13/013241 to EMD Millipore Corporation, filtration properties of nanofibers may strongly depend on the properties of the underlying support layer.

) While a filtration medium is often used in a single-layer configuration, it is sometimes advantageous to provide more than one layer of filtration medium adjacent to each other. Layering membrane filters to improve particle retention is commonly used in virus filtration and is practiced commercially in EMD Millipore Corporation's Viresolve® NFP and Viresolve Pro® product lines. Layering filtration media of the same or different composition is also used to improve filter throughput. Examples of such layered filters include EMD Millipore Corporation's Express® SHC and SHRP product lines.

Other considerations for choosing a multi-layered filtration product include economics and convenience of media and device manufacturing, ease of sterilization and validation. The fibrous filtration media of the present invention can be used in single-layer or in multi-layer configuration.

The preferred layer configuration for the filtration medium is often selected based on practical considerations. These considerations take into account the known relationship between LRV and thickness, whereby LRV typically increases with thickness. A practitioner can select multiple ways of achieving desired levels of LRV, e.g. by using fewer layers of larger thickness or larger number of thinner layers.

## VI. EXEMPLARY TEST METHODS USED

"Basis weight" was determined by ASTM D-3776, reported in $g/m^2$.

Polymer molecular weight distribution of nylon was determined using an HPLC-SEC system equipped with a Jordi xStream 500A column from Jordi Labs, Mansfield, MA, USA, and a Waters 410 Differential RI (refractive index) Detector, from Waters Corps., Milford, MA, USA, using a solvent hexafluoroisopropanol with 0.01M sodium trifluoracetate. Calibration was performed with eleven (11) PMMA (polymethyl methacrylate) standards, having a molecular weight from 202 to 903,000.

"Porosity" was calculated by dividing the basis weight of the sample in $g/m^2$ by the polymer density in $g/cm^3$, by the sample thickness in micrometers, multiplying by 100, and subtracting the resulting number from 100, i.e., porosity=100-[basis weight/(density.times.thickness).times.100].

Fiber diameter was determined as follows: A scanning electron microscope (SEM) image was taken at 60,000 times magnification of each side of a nanofiber mat sample. The diameter of ten (10) clearly distinguishable nanofibers were measured from each SEM image and recorded. Defects were not included (i.e., lumps of nanofibers, polymer drops, intersections of nanofibers). The average fiber diameter of each side of the nanofiber mat sample was calculated. The measured diameters also include a metal coating applied during sample preparations for SEM. It was established that such coating adds approximately 4 to 5 nm to the measured diameter. The diameters reported here have been corrected for this difference by subtracting 5 nm from the measured diameter.

Thickness was determined by ASTM D1777-64 and is reported in micrometers (or microns) and is represented by the symbol "$\mu$m".

Mean flow bubble point was measured according to ASTM E1294-89, "Standard Test Method for Pore Size Characteristics of Membrane Filters Using Automated Liquid Porosimeter". By using automated bubble point method according to ASTM F316 using a custom-built capillary flow porosimeter, in principle similar to a commercial apparatus from Porous Material, Inc. (PMI), Ithaca, N.Y, USA. Individual samples of 25 mm in diameter were wetted with perfluorohexane, commercially available from 3M, St. Paul, MN USA, as Fluorinert™ FC-72. Each sample was placed in a holder, and a differential pressure of air was applied and the fluid removed from the sample. The differential pressure at which wet flow is equal to one-half the dry flow (flow without wetting solvent) is used to calculate the mean flow pore size using supplied software.

"Permeability" is the rate at which fluid passes through the sample of a given area at a given pressure drop and was measured by passing deionized water through filter medium samples having a diameter of 47 (9.6 cm$^2$ filtration area) mm. The water was forced through the samples using hydraulic pressure (water head pressure) or pneumatic pressure (air pressure over water).

The "effective pore size" of an electrospun mat can be measured using conventional membrane techniques such as bubble point, liquid-liquid porometry, and challenge test with particles of certain size. It is known that the effective pore size of a fibrous mat generally increases with the fiber diameter and decreases with porosity.

"Bubble point test" provides a convenient way to measure effective pore size. It is calculated from the following equation: $P = \frac{2\gamma}{r}\cos\theta$ , where P is the bubble point pressure, $\gamma$ is the surface tension of the probe fluid, r is the pore radius, and $\theta$ is the liquid-solid contact angle. Membrane manufacturers assign nominal pore size ratings to commercial membrane filters, which usually indicate meeting certain retention criteria for particles or microorganisms rather than geometrical size of the actual pores.

VII. EXEMPLARY DETERMINATION OF PARVOVIRYS RETETION

Parvovirus retention was tested following an EMD Millipore Corporation test method, wherein the bacteriophage PhiX-174 challenge stream was prepared with a minimum titer of $1.0 \times 10^6$ pfu/mL in a 50 mM acetate buffer solution, pH 5.0, containing 100 mM NaCl. Porous media to be tested were cut into 25 mm discs and sealed in overmolded polypropylene devices. These devices were then challenged by the above provided bacteriophage PhiX-174 challenge stream at 15 psi pressure after being wet with water at 30 psi. Initial 5 ml of effluent were discarded to eliminate effects of dilution with hold-up volume. 4 ml fractions were collected immediately after the discarded 5 ml ($LRV_a$) as well as at the end of the run after 200 ml of effluent ($LRV_{final}$). Quantification of bacteriophage in the initial and final feed were conducted on plates incubated overnight using a light box and a colony counter. Corresponding log retention values (LRV) were calculated.

The following Examples of different embodiments of the present invention will demonstrate that an electrospun nanofiber mat can simultaneously possess both high permeability and high parvovirus retention.

Examples

Example 1. Preparation of nylon stock solution for electrospinning

This example provides an exemplary procedure for preparing a nylon solution for electrospinning in accordance with an embodiment of this invention.

Nylon 6 was supplied by BASF Corp., Florham Park. NJ, USA. under the trademark Ultramid 1124, A 15% wt. solution was prepared in a mixture of three solvents: formic acid, acetic acid and water, present in weight ratio 2:2:1. The solution was prepared by vigorously stirring the mixture of solvents and polymer in a glass reactor for 5 to 6 hours at 80° C. It was subsequently cooled to room temperature. The molecular weight of the final polymer was analyzed by SEC and found to be reduced as a result of heating in this solvent system (Table 1). In addition, the molecular weight distribution (Mw/Mn) was also reduced.

Table 1. Molecular weight analysis of nylon 6 before preparation of stock solution and as a result of hydrolysis.

| Time | Mw | Mw/Mn | Mn |
|---|---|---|---|
| 0 | 28,503 | 3,41 | 8,355 |
| 5 hours | 23,282 | 2,36 | 9,861 |
| 24 hours | 14,134 | 1,93 | 7,307 |

It was found that reducing the molecular weight of the polymer (e.g.. nylon) through hydrolysis results in the formation of nanofibers having a smaller diameter.

Example 2. Preparation of nylon solution ready for electrospinning in accordance with an embodiment of this invention.

The stock solution prepared in Example 1 was diluted to 8% wt. polymer with 2.2,2-trifluroethanol (TFE), formic acid, and water. Ammonium formate is added to the concentration of 1% wt. The composition of the spinning solution is listed in Table 2.

Table 2. Composition of spinning solution.

|  | % by weight |
| --- | --- |
| nylon 6 | 8 |
| Formic Acid | 20.5 |
| Acetic Acid | 20.5 |
| Water | 10.3 |
| 2.2,2-Trifluoroethanol | 40 |
| Ammonium Formate | 0.7 |

Viscosity of the final solution was 30 to 35 cP at 25° C, and the conductivity was 1.0 to 1.5 mS/cm.

Example 3. Preparation of a parvovirus-retentive mat in accordance with an embodiment of this invention.

The solution from Example 2 was immediately spun using a Nanospider™ nozzle-free electrospinning apparatus, available from Elmarco, Inc., Morrisville, NC USA . The 6-wire rotating electrode is equipped 33-gauge steel wire, distance between solution pan and collector is 140 mm, electrode rotation speed is 60 Hz, humidity is maintained between 10° and 16° dew point using an external humidification system. Spinning time is 30 min. In this embodiment, the supporting material used to collect the nanofibers is also an electrospun nylon 6 mat with an average fiber diameter of about 100 nm. In this embodiment the supporting electrospun material is produced by electrospinning nylon 6 from a 12wt. % solution in a mixture of acetic and formic acids, with weight ratio between the acids being 2: 1, using the same electrospinning equipment and parameters provided *supra*, however generally without controlling humidity.

Table 3 outlines the properties of the electrospun mat produced, including a key measure of the pore size as indicated by the mean flow bubble point, measured with a low surface tension fluid (-10 dynes/cm). Fluorinert™ FC-72. available from 3M.

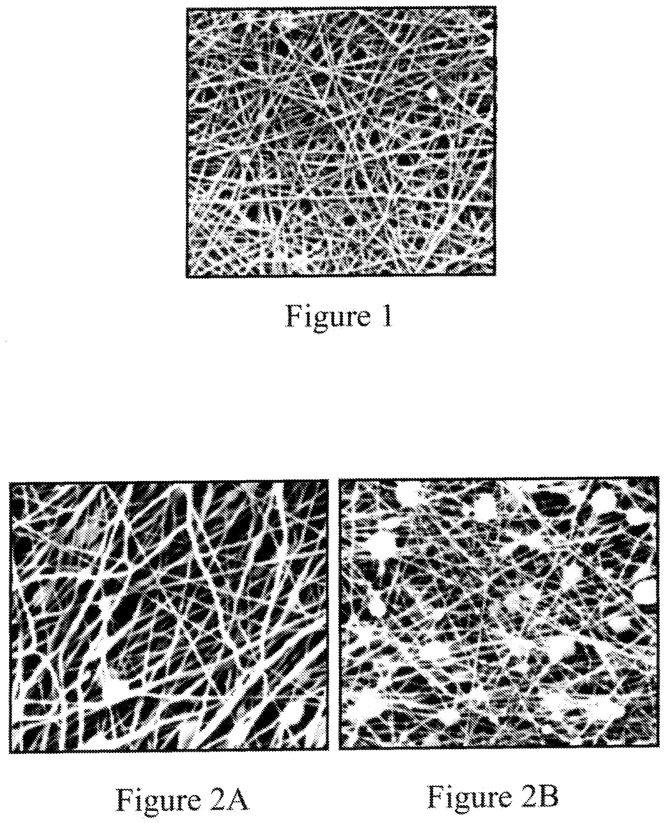

Figure 1 shows a SEM micrograph of an electrospun mat according to one embodiment of the invention, having a measured average fiber diameter, after correction for the SEM coating layer, of about 9-12 nm. The bubble point values for this embodiment of the invention clearly indicate that the electrospun mats exhibit very small pore sizes, in the range of traditional parvovirus retentive membranes, which usually fall in the range between 120 psi and 180 psi.

Table 3.

| Fiber diameter (nm) | 9-12 nm |
| --- | --- |
| FC-72 bubble point, initial (psi) | 131 |
| FC-72 bubble point, mean flow (psi) | 136 |
| Air permeability for a 0.45cm$^2$ disk (scfm/psi) | 0.00496 |
| Thickness of the nanofiber layer with small fiber diameter (microns) | 16 |

Comparative Example 1, Effect of humidity on nanofiber mat quality

Comparative Example I demonstrates that maintaining humidity above approximately 10° C dew point is advantageous for producing smaller diameter nanofiber mats. A 12% solution of nylon 6 was prepared in a mixture of formic acid, acetic acid, and water, present in weight ratio 2:2:1, by heating the mixture at 80° C for 10 hours. The solution was cooled and nanofiber mats were spun using the Elmarco Lab Nanospider™ nozzle-free electrospinning apparatus used in Example 3.

It can be seen from Figure 2 that increasing the humidity from 4° C dew point to 17° C dew point reduces the fiber diameter, from 23 nm to 9 nm on average. The electrospun mat produced at a higher humidity, however, cannot be used in filtration due obvious beading. Beading creates significant defects in the nanofiber mat structure, resulting in poor mechanical strength and broad pore size distribution. Therefore, rather than using increased humidity to produce smaller diameter nanofiber mats, other improvements are necessary to create a quality nanofiber mat with small diameters.

Example 2.

Example 2 demonstrates that the addition of 2.2,2-triflutoethanol (TFE) to a spinning solution substantially improves the quality of the nanofiber mats produced. An electrospinning stock solution was prepared according to Example 1. Two equal fractions of the stock solution were taken.

Fraction A was further diluted to 8wt.% polymer (i.e., nylon 6) concentration with the same solvent mixture as used for stock solution (formic acid, acetic acid, water).

Fraction B was diluted with a mixture of four solvents: formic acid, acetic acid, water, and TFE, to a final TFE concentration of 25%wt.

Ammonium formate was added to both solutions to a concentration 0.5%wt.

Solution A: viscosity 32 cP, conductivity 3.4 mS/cm.

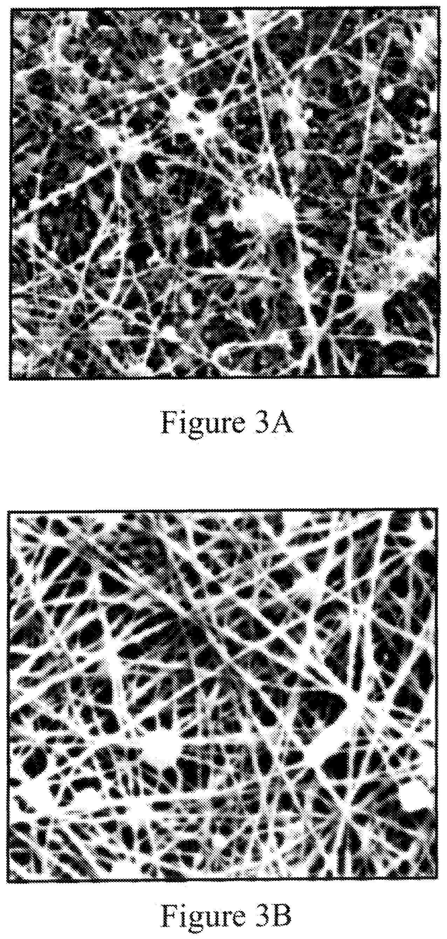

Solution 13: viscosity 35 cP, conductivity 2.6 mS/cm. SEM micrographs of the mats produced with these solutions are shown in Figure 3. It can be seen that mat produced with TFE has much greater consistency of fibers and less fiber breakage.

Comparative Example 2. Reduction of nylon 6 concentration in spinning solution

Comparative Example 2 demonstrates that reducing the nylon 6 concentration to 8% without using ammonium formate or TFE in the mix does not result in the formation of a consistent electrospun mat with a small fiber diameter.

A stock solution of nylon 6 was prepared according to Example 1, and subsequently diluted with the same solvent mixture (formic acid, acetic acid, and water) and used for electrospinning. Table 4 lists properties of solutions and average fiber diameters of the electrospun mats.

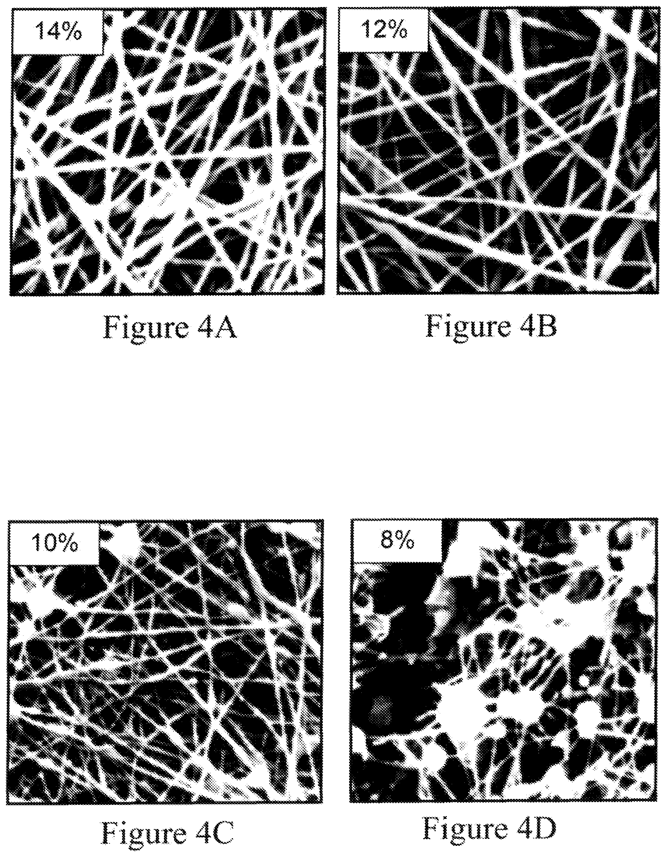

Figure 4 demonstrates that lower concentrations of nylon result in highly irregular mats.

Table 4. Solution properties of nylon 6 and resulting electrospun mat properties

| Concentration (%wt.) | Solution viscosity (cP) | Conductivity (mS/cm) | Fiber diameter (nm) | Mat quality |
|---|---|---|---|---|
| 8 | 26 | 1.026 | 11 | Very poor |
| 10 | 48 | 1.084 | 15 | Fair |
| 12 | 89 | 1.04 | 29 | Good |
| 14 | 114 | 1.01 | 40 | Good |

Comparative example 3. Optimization of spinning solution.

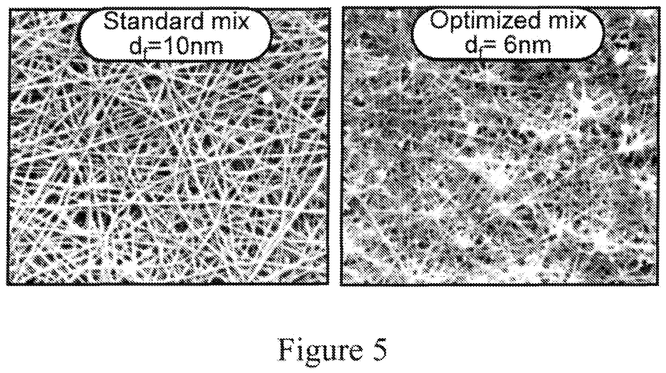

Solution properties were further optimized to result in an electrospun nanofiber mat with the smallest fibers, which is also homogeneous and integral. Table 5 displays the optimized solution properties and Figure 5 presents the SEM comparison of the 6nm fibers with the 10nm fibers. The polymer solution described below had a viscosity of 24cP and a conductivity of 3.68 mS/cm.

Table 5. Composition of spinning solution

|  | Mix components by weight (%) |
|---|---|
| Nylon 6 | 7 |
| Formic Acid | 20.8 |
| Acetic Acid | 20.8 |
| Water | 10.4 |
| 2,2,2-Trifluoroethanol | 40 |
| Ammonium Formate | 1 |

Example 4. Retention of model parvovirus in buffer solution

OptiScale-25 devices. EMD Millipore Corporation, Billerica, MA USA, were manufactured containing three (3) layers of electrospun composites having a filtration area of 3.5 cm$^2$. The layers were interleaved with a polypropylene non-woven fabric.

Water permeability and virus retention were tested in a constant pressure setup equipped with a load cell. Model virus, bacteriophage PhiX-174, was spiked into acetate buffer at pH 5, conductivity 13.5 mS/cm, to concentration $1.4*10^7$ PFU/ml. The devices were flushed with buffer for 10 min, feed was switched to the virus-spiked vessel, and 200 mL of spiked buffer was flowed through each device. Samples for virus assays were collected after 5 mL, 60 mL, 130 mL, and 200 mL.

Table 6 lists buffer permeability of the devices, and Figure 5 shows LRV as an average of two (2) devices.

Table 6. Device flow rates and permeabilities with buffer fluxes.

|  | Device flow rate (mL/min) | Permeability (LMH/psi) |
|---|---|---|
| 3-layer – 3.5 cm2 | 4.5 at 15 psi | 52 |
| Viresolve Pro – 3.1 cm2 | 2.3 at 30 psi | 15 |

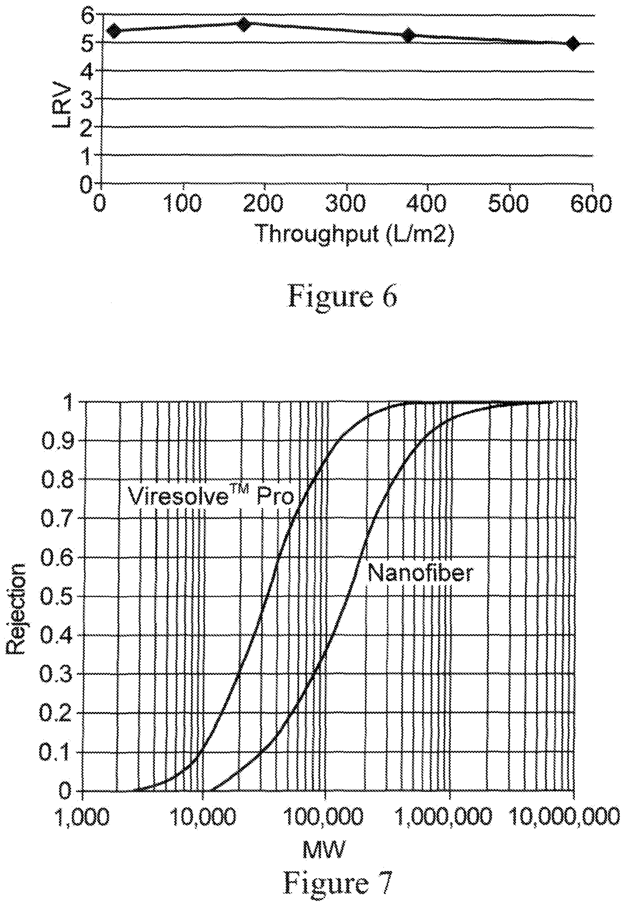

As depicted in Figure 6 both devices demonstrate high retention of model parvovirus PhiX-174, which was not significantly reduced over the course of the filtration experiment. This data, along with bubble points and fiber diameters, clearly demonstrates that parvovirus-retentive electrospun mats can be prepared. The measured permeabilities exceed the value of Viresolve™ Pro.

Comparative Example 4This example demonstrates that spinning the active layer on a smoother, smaller fiber diameter substrate results in superior filtration properties like higher retention achieved at lower thicknesses, leading to higher permeability. A solution similar to the one described in Example 2 was spun for 15 minutes at 14 °C dew point resulting in a nanofiber mat that is about 4 um thick, with an average fiber diameter of 10nm. The substrate used was also a nanofiber mat spun for 15 minutes on a smooth Hirose nonwoven substrate (Hirose Paper Manufacturing Co., Ltd, Tosa-City, Kochi, Japan, part number # HOP-60HCF), using a 13wt% B24 grade Nylon6 mix in 2:2:1/Formic acid: Acetic acid: Water. The nanofiber substrate was about 10 um thick, with an average fiber diameter of 23nm. This extremely thin nanofibrous structure displayed superior filtration properties in a single layer format compared to the devices produced using the three layered configuration described in Example 4. Bubble point, water permeability and virus retention in buffer were tested as described in the text and the results are shown in Table 7.

Table 7. Physical and filtration properties of electrospun mats based on the substrate layer properties.

| | $d_{active}$ (nm) | $t_{active}$ (um) | $d_{subst rate}$ (nm) | $t_{subst rate}$ (um) | Mean flow BP (psi) | Number of layers in device | Perm eabili ty (lmh/ psi) | LRV initial | LRV final |
|---|---|---|---|---|---|---|---|---|---|
| Example 3 | 9 - 12 | 16 | 100 | 20 | 136 | 3 | 52 | 5.5 | 5.0 |
| Compara tive Example 3 | 10 | 4 | 23 | 10 | 131 | 1 | 127 | 6.1 | 6.0 |

Example 5. Retention of model parvovirus in a protein-containing solution

In one embodiment of the invention, nanofiber mats are produced according to Example 3, and are surface-modified to reduce potential non-specific protein binding. An aqueous solution is prepared containing 3.25%wt. of ethoxylated (20) trimethylolpropane triacrylate, commercially available as SR415 from Sartomer Co.. Exton. PA. USA; and 10%wt. 2-methyl-2, 4-pentanediol (MPD). A nanofiber sheet was wet with this solution and exposed to electron beam radiation to a total dose of 2 MRad under a nitrogen atmosphere. The sheet was rinsed in water. Nine (9) 25 mM disks were cut from the wet sheet and sealed into three (3) stainless steel holders, three (3) layers per holder, available from EMD Millipore Corporation, Billerica, MA USA. catalog No. XX45 025 00. A three (3) layer prefilter made with 0.1 $\mu$m Durapore™ also available from EMD Millipore Corporation. Billerica, MA USA. is used in front of the nanofiber device.

Polyclonal IgG, acquired from SeraCare Life Science, Milford, MA USA, was dissolved in a 25 mM Acetate buffer, having a pH 4.0, and conductivity 2.5 mS/cm. The solution was spiked with PhiX-174 bacteriophage to a final concentration of $2.5*10^7$ pfu/ml. An initial LRV sample was taken after the first 5 mL was filtered, and a final LRV sample was taken at 75% filter plugging. Results are shown in Table 8.

Table 8. Buffer permeability, protein throughput, and virus LRV for the nanofiber devices in accordance with embodiments of the invention, and Viresolve™ Pro. Data is average for three (3) nanofiber devices and the two (2) Viresolve™ Pro devices.

| | Buffer permeability (LMH/psi) | Throughput (L/m2) at V75 | Initial LRV | Final LRV |
|---|---|---|---|---|
| Viresolve Pro | 12.0 | 439 | >6.7 | >6.7 |
| Nanofiber, 3 layers | 12.2 | 556 | 5.6 | 3.6 |

Example 6. Scale-up of electrospinning mat production

Nylon 6 solution was prepared according to Example 2. A nanofiber mat was produced on a pilot scale electrospinning apparatus from Elmarco, equipped with three rotating electrodes, each 50 cm wide. The 6-wire rotating electrode is equipped with 33-gauge steel wire, the distance between the solution pan and collector is 140 mm, the electrode rotation speed is 60 Hz, ambient humidity between 10° and 16° dew point. The electrode spin rate was 6.7 to 6.9 rpm. The mixing rods were set to rotate at 58 rpm to 60 rpm to ensure good mixing of the nylon 6 solution. The top and bottom voltage was 20 kV and 60 kV, respectively. The supporting material used to collect the nanofibers is a nylon 6 electrospun mat with an average fiber diameter of 100 nm. The supporting material is produced by electrospinning nylon 6 from a 12wt. % solution in a mixture of acetic and formic acids, with weight ratio between the acids being 2:1. using the same electrospinning equipment. For a higher productivity, the nylon 6 solution was poured into all 3 pans. The line speed was set at 2 cm/min. At the start of the run, only the pan closest to the beginning of the line is active. After 15 minutes (corresponding to 30 cm of mat length), the instrument was turned off to load the second pan. The instrument was then turned on with both first and second pans active. After another 15 min of operation, the instrument was turned off to load the third pan. The instrument was then turned on with all three pans active. The run was continued for another 45 mins (total time 75 mins) to create a sheet with an active area of 0.9 m long by 0.57 m wide. When the pans were not in use, they were covered with a lid to prevent solution changes due to high volatility of TFE. 20 mm discs were cut out from the four (4) corners, four (4) edges at the center, and one (1) from the middle. These discs were tested for bubble point

Table 9 outlines the properties of the electrospun mats produced in accordance with embodiments of the invention, based on the test of the representative disc samples.

In Table 9, a key measure of the pore size as indicated by the mean flow bubble point was measured with a low surface tension fluid (10 dynes/cm), Fluorinert™ FC-72 available from 3M, St. Paul, MN USA. The bubble point values indicate that the electrospun mats produced in accordance with embodiments of the invention, exhibit very small pore sizes, in the range of parvovirus retentive membranes, which usually fall in the range between 120 and 180 psi.

Table 9

| Fiber diameter (nm) | 17 nm |
|---|---|
| FC-72 bubble point, initial (psi) (median ± 1 s.d.) | 115±14 |
| FC-72 bubble point, mean flow (psi) (median ± 1 s.d.) | 131±8 |
| Air permeability for a 0.45cm$^2$ disk (scfm/psi) | 0.0082±0.001 |

Example 7.

Characterization by dextran sieving measurements

Nanofiber mats produced according to Example 6 were characterized using a dextran retention test. For this study, two (2) 44.5 mm diameter nanofiber discs (along with the non-woven support used to spin the mat on) were cut from the mat. Two (2) 44.5 mm discs of VireSolve™ Pro were used as controls. The discs were submerged in water, and then placed in a stirred cell available from EMD Millipore (catalog no. 5122). 40 ml of a mixture of various dextran sizes was poured into the stirred cell. A standard magnetic stir bar was placed in the cell. The stirred cell was then placed on a magnetic stir plate. PVC tubing 1/16" ID (Fisher scientific catalog no. 14-190-118) attached to a peristaltic pump was connected to the permeate side to draw liquid at constant flow rate.

The other end on the tube was placed into the stirred cell to allow recirculation. Dextran solution comprising various molecular weights was then poured in to the stirred cell. The peristaltic pump was then turned on and run at 0.8 ml/min. The first 2 to 3 ml was discarded before recirculation to avoid any contamination of the feed dextran solution. The pump was run for two (2) hrs to allow equilibration. After two (2) hrs of operation, the pump was turned off, and the sample in the tubing was collected for further analysis using gel permeation chromatography (GPC).

Based on the GPC results the dextran retention curve was generated for the VireSolve™ Pro and the nanofiber mats in accordance with the invention (Figure 7). The average R90 (90% dextran rejection) from two (2) discs for each of VireSolve™ Pro and the nanofiber mats were 100 KDa and 500 KDa, respectively. Although the nanofiber mat does not appear to be as tight as VireSolve™ Pro, the nanofiber mat in accordance with the invention does nonetheless demonstrate good retention in the ultrafiltration membrane range.

Example 8. Throughput and retention measurement with a solution containing cell culture bioreactor media

For throughput studies with cell culture media. OptiScale-25 devices were manufactured containing three (3) layers of electrospun composites produced according to Example 6, with a filtration area of 3.5 cm$^2$. One (1) layer of a polypropylene non-woven fabric was used downstream. Water permeability and cell culture media throughput were tested in a constant pressure setup equipped with a load cell. For these studies, CD CHO media (catalog no. 10743 - 029) from Life Technologies was used. CD CHO medium is a protein-free, serum-free, chemically-defined medium optimized for the growth of Chinese hamster ovary (CHO) cells and expression of recombinant proteins in suspension culture.

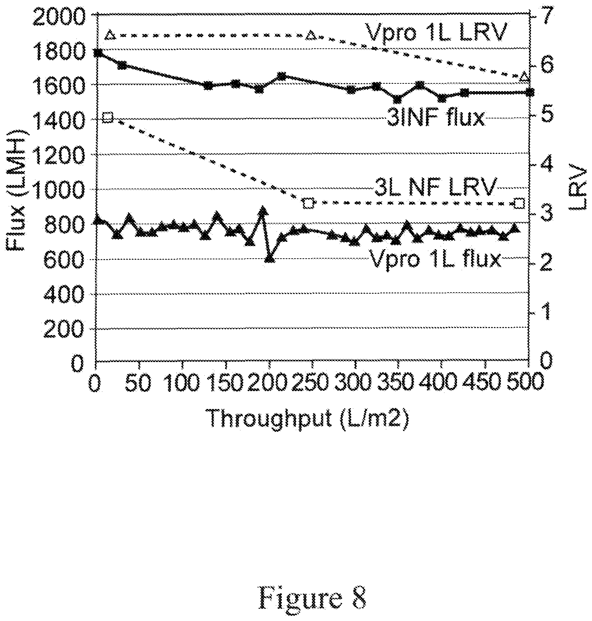

For virus retention studies with cell culture media, OptiScale-25 devices were manufactured containing three (3) layers of electrospun composites produced according to Example 6, with a filtration area of 3.5 cm$^2$. One (1) layer of a polypropylene non-woven fabric was used at the bottom (outlet) side for the device. Water permeability and virus retention were tested in a constant pressure setup equipped with a load cell. Model virus, bacteriophage PhiX-174, was spiked into CD CHO cell culture media to concentration 1.4*10$^7$ PFU/ml. The devices were flushed with water for 10 mins, feed was switched to the virus-spiked vessel, and virus-spiked media was flowed through each device. Samples for virus assays were collected at various throughputs (15, 250, and 500 L/m$^2$). Figure 8 shows LRV and flux decay as an average of two (2) devices. Also shown is one (1) layer of Viresolve™ Pro membrane. All devices were integrity tested post-use to detect gross leaks by monitoring air bubbles downstream from the devices by pressurizing the devices at 50 psi. Only devices that had <5 bubbles per minute were chosen as integral. The nanofiber composite gives >3 LRV retention up to 500 L/m$^2$ with 2X the permeability of one (1) layer Viresolve™ Pro membrane. If higher virus retention is necessary, it is possible to increase the number of layers to give additional virus retention. This example indicates that nanofiber composites can be used for virus removal from cell culture media.

Unless otherwise indicated, all numbers expressing quantities of ingredients, cell culture, treatment conditions, and so forth used in the specification, including claims, are to be understood as being modified in all instances by the term "about". Accordingly, unless otherwise indicated to the contrary, the numerical parameters are approximations and may vary depending upon the desired properties sought to be obtained by the present invention. Unless otherwise indicated, the term "at least" preceding a series of elements is to be understood to refer to every element in the series..

Many modifications and variations of this invention can be made without departing from its scope, as will be apparent to those skilled in the art. The specific embodiments described herein are offered by way of example only and are not meant to be limiting in any way. It is intended that the specification and examples be considered as exemplary only, with the true scope of the invention being indicated by the following claims.

**Claims**

1. A fibrous electrospun porous media for removing viral particles and other particles in the 18 nm to 30 nm size range from an aqueous fluid stream comprising electrospun nanofibers formed from nylon-6, nylon-6,6, nylon 6,6-6,10, nylon-6 copolymers, nylon-6,6 copolymers, nylon 6,6-6,10 copolymers or mixtures thereof and having an average fiber diameter of 6 nm to 13nm, the media having a mean flow pore size ranging from 0.01 $\mu$m to 0.03 $\mu$m wherein the mean flow pore size is measured by the method disclosed herein,an average thickness of 1 $\mu$m to 100 $\mu$m as determined by ASTM D1777-64, a liquid permeability greater than 10 LMH/psi and a porosity ranging from 80% to 95%;

   wherein the media is a liquid filtration mat;
   wherein the porosity is calculated by the method disclosed herein;
   wherein the fiber diameter is calculated by the method disclosed herein; and
   wherein the pore size is measured using the equation $P = \frac{2\gamma}{r} cos\theta$ , where P is the bubble point pressure, $\gamma$ is the surface tension of the probe fluid, r is the pore radius, and $\theta$ is the liquid-solid contact angle.

2. The media according to claim 1, the thickness ranging from 2 $\mu$m to 30 $\mu$m.

3. The media according to claim 1, wherein the electrospun porous media has a mean flow bubble point measured with perfluorohexane that is above 120 psi (827 kPa).

4. A liquid filtration device for removing viral particles in the 18 nm to 30 nm size range from an aqueous feed solution, the device comprising:

   a fibrous electrospun porous media according to any one of claims 1 to 3, and a porous support,
   wherein the fibrous electrospun porous media is disposed on the porous support.

5. The device according to claim 4, wherein the porous support is a nanofibrous support layer with average fiber diameter between 10 nm and 500 nm.

6. The device according to claim 4, wherein the porous support is a nanofibrous support layer having average fiber diameters between 50 nm and 200 nm.

7. The device according to claim 4, wherein the porous support is a nanofibrous support layer having average fiber diameters between 10 nm and 50 nm.

8. A method of removing virus contaminants and other particles in the 18 nm to 30 nm size range from an aqueous fluid feed solution comprising the steps of:

   providing a fibrous electrospun porous media according to any one of claims 1 to 3, and a porous support, wherein the fibrous electrospun porous media is disposed on the porous support;
   contacting the virus contaminated aqueous fluid feed solution with the fibrous electrospun porous media and porous support; and
   obtaining a filtrate having less than 0.01% of virus contaminants present in the aqueous fluid feed solution.

9. The method according to claim 8, wherein the filtrate has:

   a) less than 0.001% of viruses present in the feed solution; or preferably
   b) less than 0.0001% of viruses present in the feed solution.

10. The method according to claim 8 or claim 9, wherein the virus is a parvovirus.


**Patentansprüche**

1. Faseriges elektrogesponnenes poröses Medium zum Entfernen von Viruspartikeln und anderen Partikeln in dem Größenbereich von 18 nm bis 30 nm von einem wässrigen Fluidstrom, umfassend elektrogesponnene Nanofasem, die aus Nylon-6, Nylon-6,6, Nylon-6,6-6,10, Nylon-6-Copolymeren, Nylon-6,6-Copolymeren, Nylon-6,6-6,10-Copolymeren oder Mischungen davon ausgebildet sind und einen durchschnittlichen Faserdurchmesser von 6 nm bis 13 nm aufweisen, wobei das Medium eine mittlere Fließporengröße in einem Bereich von 0,01 $\mu$m bis 0,03 $\mu$m, wobei die mittlere Fließporengröße durch das hierin offenbarte Verfahren gemessen wird, eine durchschnittliche Dicke von 1 $\mu$m bis 100 $\mu$m wie durch ASTM D1777-64 bestimmt, eine Flüssigkeitspermeabilität von mehr als 10 LMH/psi und eine Porosität in einem Bereich von 80 % bis 95 % aufweist;

   wobei das Medium eine Flüssigkeitsfiltrationsmatte ist;
   wobei die Porosität durch das hierin offenbarte Verfahren berechnet wird;
   wobei der Faserdurchmesser durch das hierin offenbarte Verfahren berechnet wird; und
   wobei die Porengröße unter Verwendung der Gleichung $P = \frac{2\gamma}{r} cos\theta$ gemessen wird, wobei P der Blasenpunktdruck ist, $\gamma$ die Oberflächenspannung des Sondenfluids ist, r der Porenradius ist und $\theta$ der Flüssigkeit-Feststoff-Kontaktwinkel ist.

2. Medium nach Anspruch 1, wobei die Dicke in einem Bereich von 2 $\mu$m bis 30 $\mu$m liegt.

3. Medium nach Anspruch 1, wobei das elektrogesponnene poröse Medium einen mittleren Fließblasenpunkt aufweist, gemessen mit Perfluorhexan, der über 120 psi (827 kPa) liegt.

4. Flüssigkeitsfiltrationsvorrichtung zum Entfernen von Viruspartikeln in dem Größenbereich von 18 nm bis 30 nm von einer wässrigen Zufuhrlösung, die Vorrichtung umfassend:

   ein faseriges elektrogesponnenes poröses Medium nach einem der Ansprüche 1 bis 3 und einen porösen Träger, wobei das faserige elektrogesponnene poröse Medium auf dem porösen Träger angeordnet ist.

5. Vorrichtung nach Anspruch 4, wobei der poröse Träger eine nanofaserige Trägerschicht mit einem durchschnittlichen Faserdurchmesser zwischen 10 nm und 500 nm ist.

6. Vorrichtung nach Anspruch 4, wobei der poröse Träger eine nanofaserige Trägerschicht ist, die durchschnittliche Faserdurchmesser zwischen 50 nm und 200 nm aufweist.

**7.** Vorrichtung nach Anspruch 4, wobei der poröse Träger eine nanofaserige Trägerschicht ist, die durchschnittliche Faserdurchmesser zwischen 10 nm und 50 nm aufweist.

**8.** Verfahren zum Entfernen von Virusverunreinigungen und anderen Partikeln in dem Größenbereich von 18 nm bis 30 nm von einer wässrigen Fluidzufuhrlösung, umfassend die Schritte:

> Bereitstellen eines faserigen elektrogesponnenen porösen Mediums nach einem der Ansprüche 1 bis 3 und eines porösen Trägers, wobei das faserige elektrogesponnene poröse Medium auf dem porösen Träger angeordnet ist;
> Inberührungbringen der virusverunreinigten wässrigen Fluidzufuhrlösung mit dem faserigen elektrogesponnenen porösen Medium und dem porösen Träger, und
> Erhalten eines Filtrats, das weniger als 0,01 % der Virusverunreinigungen aufweist, die in der wässrigen Fluidzufuhrlösung vorhanden sind.

**9.** Verfahren nach Anspruch 8, wobei das Filtrat aufweist:

> a) weniger als 0,001 % von Viren, die in der Zufuhrlösung vorhanden sind; oder vorzugsweise
> b) weniger als 0,0001 % von Viren, die in der Zufuhrlösung vorhanden sind.

**10.** Verfahren nach Anspruch 8 oder 9, wobei das Virus ein Parvovirus ist.

**Revendications**

**1.** Milieu poreux fibreux électrofilé permettant de retirer des particules virales et d'autres particules dans la plage de taille de 18 nm à 30 nm d'un courant de fluide aqueux comprenant des nanofibres électrofilées formées de nylon-6, de nylon-6,6, de nylon 6,6-6,10, de copolymères de nylon-6, de copolymères de nylon-6,6, de copolymères de nylon 6,6-6,10 ou de mélanges de ceux-ci et ayant un diamètre moyen de fibre de 6 nm à 13 nm, le milieu ayant une taille moyenne de pore d'écoulement allant de 0,01 $\mu$m à 0,03 $\mu$m dans lequel la taille moyenne de pore d'écoulement est mesurée par le procédé décrit ici, une épaisseur moyenne de 1 $\mu$m à 100 $\mu$m telle que déterminée par ASTM D1777-64, une perméabilité aux liquides supérieure à 10 LMH/psi et une porosité allant de 80 % à 95 % ;

> dans lequel le milieu est un tapis de filtration de liquides ;
> dans lequel la porosité est calculée par le procédé décrit ici ;
> dans lequel le diamètre de fibre est calculé par le procédé décrit ici ; et
>
> dans lequel la taille de pore est mesurée à l'aide de l'équation $P = \frac{2\gamma}{r} cos\theta$ , où P est la pression de point de bulle, $\gamma$ est la tension superficielle du fluide de sonde, r est le rayon de pore, et $\theta$ est l'angle de contact liquide-solide.

**2.** Milieu selon la revendication 1, l'épaisseur allant de 2 $\mu$m à 30 $\mu$m.

**3.** Milieu selon la revendication 1, dans lequel le milieu poreux électrofilé a un point de bulle d'écoulement moyen mesuré avec du perfluorohexane qui est supérieur à 120 psi (827 kPa).

**4.** Dispositif de filtration de liquide destiné à retirer des particules virales dans la plage de taille de 18 nm à 30 nm d'une solution d'alimentation aqueuse, le dispositif comprenant :

> un milieu poreux fibreux électrofilé selon l'une quelconque des revendications 1 à 3, et un support poreux,
> dans lequel le milieu poreux fibreux électrofilé est disposé sur le support poreux.

**5.** Dispositif selon la revendication 4, dans lequel le support poreux est une couche de support nanofibreuse avec un diamètre moyen de fibre entre 10 nm et 500 nm.

**6.** Dispositif selon la revendication 4, dans lequel le support poreux est une couche de support nanofibreuse ayant des diamètres moyens de fibre entre 50 nm et 200 nm.

**7.** Dispositif selon la revendication 4, dans lequel le support poreux est une couche de support nanofibreuse ayant des diamètres moyens de fibre entre 10 nm et 50 nm.

8. Procédé permettant de retirer des contaminants viraux et d'autres particules dans la plage de taille de 18 nm à 30 nm d'une solution d'alimentation fluide aqueuse comprenant les étapes consistant à :

fournir un milieu poreux fibreux électrofilé selon l'une quelconque des revendications 1 à 3, et un support poreux, dans lequel le milieu poreux fibreux électrofilé est disposé sur le support poreux ;
mettre en contact la solution d'alimentation fluide aqueuse à contamination virale avec le milieu poreux fibreux électrofilé et le support poreux ; et
obtenir un filtrat ayant moins de 0,01 % de contaminants viraux présents dans la solution d'alimentation fluide aqueuse.

9. Procédé selon la revendication 8, dans lequel le filtrat a :

a) moins de 0,001 % de virus présents dans la solution d'alimentation ; ou de préférence
b) moins de 0,0001 % de virus présents dans la solution d'alimentation.

10. Procédé selon la revendication 8 ou la revendication 9, dans lequel le virus est un parvovirus.

Figure 1

Figure 2A

Figure 2B

Figure 3A

Figure 3B

Figure 4A

Figure 4B

Figure 4C

Figure 4D

Figure 5

Figure 6

Figure 7

Figure 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2010107503 A1
- US 20120125847 A1
- US 20060016748 A1
- US 20120061332 A1
- US 20080J64214 A1
- US 6770204 B
- US 7927885 B
- US 20080264259 A
- WO 2008073507 A
- WO 2010107503 A
- WO 2012021308 A
- US IJS20110198282 A
- US 20080264258 A
- US 20040038014 A
- US 20050163955 A
- US 20040038013 A
- WO 2009071909 A
- US 7790135 B
- WO 2005024101 A
- WO 2006131081 A
- WO 2008106903 A
- WO 2013013241 A

**Non-patent literature cited in the description**

- Ensuring Safety of Biopharmaceuticals: Virus and Prion Safety Considerations. **H. ARANHA.** Filtration and Purification in the Biopharmaceutical Industry. Informa Healthcare, 2008
- **LEPPARD, KEITH ; NIGEL DIMMOCK ; EASTON, ANDREW.** Introduction to Modem Virology. Blackwell Publishing Limited, 2007, 450
- **WANG et al.** Electrospun nanofibrous membranes for high flux microfiltration. *Journal of Membrane Science,* 2012, vol. 392-393, 167-174
- **MA et al.** *J.Mater. Chem.,* 2011, vol. 21, 7507-7510
- **HUANG et al.** *Nanotechnology,* 2006, vol. 17, 1558-1563
- **TAN et al.** *Polymer,* 2005, vol. 46, 6128-6134
- **HOU et al.** *Macromolecules,* 2002, vol. 35, 2429-2431
- **DUAN et al.** *2nd IEEE International Nanoelectronics Conference,* 2008, 33-38
- **LEPPARD, KEITH ; NIGEL DIMMOCK ; EASTON, ANDREW.** Introduction to Modern Virology. Blackwell Publishing Limited, 2007, 450